# EUROPÄISCHE PATENTSCHRIFT

(11) **EP 1 341 770 B1**
(45) Veröffentlichungstag und Bekanntmachung des Hinweises auf die Patenterteilung: **14.06.2006**
(21) Anmeldenummer: 01999563.8
(22) Anmeldetag: 22.11.2001
(51) Int. Cl.: C07D 235/30, A61K 31/4184, A61P 11/00

(54) **SUBSTITUIERTE 2-ANILINO-BENZIMIDAZOLE UND IHRE VERWENDUNG ALS NHE-INHIBITOREN**
SUBSTITUTED 2-ANILINO-BENZIMIDAZOLES AND THE USE THEREOF AS NHE-INHIBITORS
2-ANILINO-BENZIMIDAZOLES SUBSTITUES ET LEUR UTILISATION EN TANT QU'INHIBITEURS DE NHE

(30) Priorität: 05.12.2000 DE 10060292
(43) Veröffentlichungstag der Anmeldung: 10.09.2003
(73) Patentinhaber: Sanofi-Aventis Deutschland GmbH, 65929 Frankfurt am Main (DE)
(72) Erfinder: HOFMEISTER, Armin, 55283 Nierstein (DE); HEINELT, Uwe, 65187 Wiesbaden (DE); LANG, Hans-Jochen, 65719 Hofheim (DE); BLEICH, Markus, 65597 Hünfelden-Dauborn (DE); WIRTH, Klaus, 65830 Kriftel (DE); GEKLE, Michael, 97072 Würzburg (DE)
(86) Internationale Anmeldenummer: PCT/EP2001/013586
(87) Internationale Veröffentlichungsnummer: WO 2002/046169

(56) Entgegenhaltungen:
- EP-A- 0 640 588
- WO-A-97/24113
- OMAR A-M M E: "The cyclodesulfurization of thio compounds; VII. A new facile synthesis of N(alpha)-substituted benzimidazoles" SYNTHESIS, Nr. 1, Januar 1974 (1974-01), Seiten 41-42, XP002192342 in der Anmeldung erwähnt
- JEN T ET AL: "Amidines and related compounds. 6. Studies on structure-activity relationships of antihypertensive and antisecretory agents related to clonidine" JOURNAL OF MEDICINAL CHEMISTRY, Bd. 18, Nr. 1, 1975, Seiten 90-99, XP000567062 in der Anmeldung erwähnt
- DATABASE CAPLUS [Online] CHEMICAL ABSTRACTS SERVICE, COLUMBUS, OHIO, US; Database accession no. 1975:541756 XP002192343 & MED. PARAZITOL. PARAZIT. BOLEZNI, Bd. 44, Nr. 3, 1975, Seiten 316-322,
- TUNÇBILEK M ET AL: "Synthesis and antimicrobial activity of some new anilino benzimidazoles" ARCHIV DER PHARMAZIE - PHARMACEUTICAL AND MEDICINAL CHEMISTRY, Bd. 330, Nr. 12, Dezember 1997 (1997-12), Seiten 372-376, XP001064267

## Beschreibung

Die Erfindung betrifft substituierte Benzimidazole der Formel I 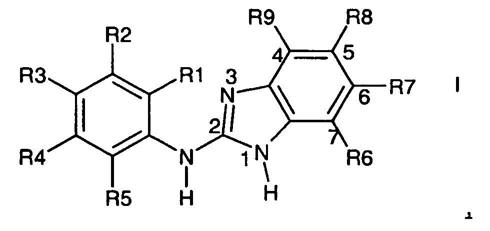 die sind
2-(2,6-Dichloro-phenylamino)-1H-benzimidazol-4-ol;
(1H-Benzimidazol-2-yl)-(2-chlor-6-methyl-phenyl)-amin;
(2,6-Dichlor-phenyl)-(5,6-difluor-1H-Benzimidazol-2-yl)-amin;
(2,6-Dichlor-phenyl)-(4-methyl-1H-Benzimidazol-2-yl)-amin;
(1H-Benzimidazol-2-yl)-(2-chlor-6-fluor-phenyl)-amin;
(1H-Benzimidazol-2-yl)-(2,6-dibrom-phenyl)-amin;
2-(2,6-Dichlorphenylamino)-5-fluorbenzimidazol;
2-(2,6-Dichlorphenylamino)-4-fluorbenzimidazol;
2-(2-Trifluormethyl-6-chlorphenylamino)benzimidazol;
2-(2,6-Dichlorphenylamino)-4,5-difluorbenzimidazol;
2-(2,6-Dichlorphenylamino)-5-hydroxybenzimidazol;
2-(2,6-Dichlorphenylamino)-4,5,6,7-tetrafluorbenzimidazol;
2-(2,6-Dichlorphenylamino)-4,6-difluorbenzimidazol;
(1H-Benzimidazol-2-yl)-(2-trifluormethyl-phenyl)-amin; oder
(1H-Benzimidazol-2-yl)-(2-brom-phenyl)-amin;
und ihre pharmazeutisch verträglichen Salze oder Trifluoracetate.

Desweiteren umfasst die Erfindung die Verwendung substituierter Benzimidazole der Formel I zur Herstellung eines Medikaments zur Behandlung von Krankheiten, welche vom NHE3-Austausch-Inhibitor beeinflusst werden, worin bedeuten:
- R1 und R5: unabhängig voneinander H, F, Cl, Br, I, CN, Alkyl mit 1 bis 4 C-Atomen,
welches unsubstituiert ist oder teilweise oder vollständig mit Fluor substituiert ist,
oder
- R1 und R5: Cycloalkyl mit 3 bis 7 C-Atomen,
welches unsubstituiert ist oder teilweise oder vollständig mit Fluor substituiert ist,
oder
- R1 und R5: OH, O-Alkyl mit 1 bis 4 C-Atomen,
welches unsubstituiert ist oder teilweise oder vollständig mit Fluor substituiert ist,
oder
- R1 und R5: OCOR10, NR11 R12, COR13, COOH, COOR14, CONR11 R12, -(O)n SOₘR15,
n Null oder 1;
m Null, 1 oder 2;
oder
- R1 und R5: O-Phenyl, welches unsubstituiert ist oder substituiert mit 1 - 3 Substituenten ausgewählt aus der Gruppe bestehend aus F, Cl, Br, J, Alkyl mit 1 bis 4 C-Atomen, OH, O-Alkyl mit 1 bis 4 C-Atomen, NR16R17, CN oder (C₁-C₄)- Alkylsulfonyl,
welches unsubstituiert ist oder teilweise oder ganz fluoriert,
R16 und R17 H oder Alkyl mit 1 bis 4 C-Atomen,
wobei die Alkylgruppen unsubstituiert sind oder teilweise oder ganz fluoriert,
R10 H oder Alkyl mit 1 bis 4 C-Atomen,
welches unsubstituiert ist oder teilweise oder vollständig mit Fluor substituiert ist,
R11 und R12 unabhängig voneinander H, Alkyl mit 1 bis 4 C-Atomen, die teilweise oder vollständig fluoriert sein dürfen und eine beliebige CH₂-Gruppe durch O oder NR18 ersetzt sein darf
oder R11 und R12 gemeinsam einen 5-, 6- oder 7-gliedrigen Ring;
oder R11 und R12 COR19 oder SO₂R20;
R18, R19 und R20 unabhängig voneinander H oder Alkyl mit 1 bis 4 C-Atomen,
welches unsubstituiert ist oder teilweise oder vollständig mit Fluor substituiert ist;
R13 und R14 Alkyl mit 1 bis 4 C-Atomen,
welches unsubstituiert ist oder teilweise oder vollständig mit Fluor substituiert ist;
R15 Alkyl oder O-Alkyl mit 1 bis 4 C-Atomen,
wobei die Alkylgruppen unsubstituiert sind oder teilweise oder ganz fluoriert, oder
R15 OH oder NR21R22;
R21 und R22 unabhängig voneinander H oder Alkyl mit 1 bis 4 C-Atomen, welches unsubstituiert ist oder teilweise oder vollständig mit Fluor substituiert ist, worin eine beliebige CH₂-Gruppe durchO oder NR23 ersetzt sein kann;
R23 H oder Alkyl mit 1 bis 4 C-Atomen,
welches unsubstituiert ist oder teilweise oder vollständig mit Fluor substituiert ist; oder
R21 und R22 gemeinsam einen 5-, 6- oder 7-gliedrigen Ring bilden;
- R2, R3 und R4: H oder F;
- R6, R7, R8 und R9: unabhängig voneinander H, F, Cl, Br, I, CN, Alkyl oder O-Alkyl mit 1 bis 4 C-Atomen,
die unsubstituiert oder ganz oder teilweise durch Fluor substituiert sind,
- oder R6, R7, R8 und R9: Cycloalkyl mit 3 bis 7 C-Atomen,
das unsubstituiert oder ganz oder teilweise durch Fluor substituiert ist,
- oder R6, R7, R8 und R9: OH, OCOR24 oder NR25R26;
R24 H oder Alkyl mit 1 bis 4 C-Atomen,
das unsubstituiert oder ganz oder teilweise durch Fluor substituiert ist;
R25 und R26 unabhängig voneinander H oder Alkyl mit 1 bis 4 C-Atomen,
das unsubstituiert oder ganz oder teilweise durch Fluor substituiert ist, oder
R25 und R26 COR27;
oder R25 und R26 gemeinsam mit dem Stickstoffatom, an das sie gebunden sind, einen 5-, 6- oder 7-gliedrigen Ring bilden, in welchem eine beliebige CH₂-Gruppe durch O oder NR18 ersetzt sein kann;
R27 H oder Alkyl mit 1 bis 4 C-Atomen,
das unsubstituiert oder ganz oder teilweise durch Fluor substituiert ist,
sowie von deren pharmazeutisch verträglichen Salzen.

Bevorzugt ist die Verwendung von Verbindungen der Formel I, worin bedeuten
- R1 und R5: unabhängig voneinander H, F, Cl, Br, CN, Alkyl mit 1 bis 4 C-Atomen, CF₃, CH₂CF₃, CF₂CF₃, Cycloalkyl mit 3 bis 7 C-Atomen, O-Alkyl mit 1 bis 4 C-Atomen, OH, OCF₃, OCH₂CF₃, OCF₂CF₃, OCOR10, NR11R12, COR13, COOH, COOR14, CONR11R12, -Oₘ-SO₂R15 oder O-Phenyl;
m Null oder 1;
R10 H, Alkyl mit 1 bis 4 C-Atomen, CF₃, CH₂CF₃ oder CF₂CF₃,
R11 und R12 unabhängig voneinander H, Alkyl mit 1 bis 4 C-Atomen, CF₃, CH₂CF₃, CF₂CF₃, wobei eine beliebige CH₂-Gruppe durch O oder NR18 ersetzt sein darf und wobei R11 und R12 zusammen mit dem Stickstoff, an den sie gebunden sind, einen 5-, 6- oder 7-gliedrigen Ring bilden darf,
oder R11 und R12 COR19 oder SO₂R20;
R18, R19 und R20 unabhängig voneinander H oder Alkyl mit 1 bis 4 C-Atomen, CF₃, CH₂CF₃ oder CF₂CF₃,
R13 und R14 Alkyl mit 1 bis 4 C-Atomen, CF₃, CH₂CF₃ oder CF₂CF₃;
R15 R15 Alkyl mit 1 bis 4 C-Atomen, CF₃, CH₂CF₃, CF₂CF₃, OH, O-Alkyl, mit 1 bis 4 C-Atomen, OCF₃, OCH₂CF₃, OCF₂CF₃ oder NR21R22;
R21 und R22 unabhängig voneinander H, Alkyl mit 1 bis 4 C-Atomen, CF₃, CH₂CF₃ oder CF₂CF₃; oder
R21 und R22 gemeinsam mit dem Stickstoffatom, an das sie gebunden sind, -(CH₂)₄-, -(CH₂)₅-, -(CH₂)₂-O-(CH₂)₂- oder -(CH₂)₂₋NR30-(CH₂)₂;
R30 H, CH₃ oder CF₃; jedoch R1 und R5 nicht gleichzeitig Cl oder CH₃ sein dürfen, und wobei höchstens einer der Substituenten R1 und R5 Wasserstoff sein darf;
- R2, R3 und R4: H oder F;
- R6, R7, R8 und R9: unabhängig voneinander H, F, Cl, Br, I, CN, Alkyl mit 1 bis 4 C-Atomen, CF₃, CH₂CF₃, CF₂CF₃, Cycloalkyl mit 3 bis 7 C-Atomen, OH, O-Alkyl mit 1 bis 4 C-Atomen, OCF₃, OCH₂CF₃, OCF₂CF₃, OCOR24 oder NR25R26;
R24 H, Alkyl mit 1 bis 4 C-Atomen, CF₃, CH₂CF₃ oder CF₂CF₃;
R25 und R26 unabhängig voneinander H, Alkyl mit 1 bis 4 C-Atomen, CF₃, CH₂CF₃, CF₂CF₃, COR27; oder
R25 und R26 gemeinsam mit dem Stickstoff, an den sie gebunden sind, einen 5-, 6- oder 7-gliedrigen Ring;
R27 H, Alkyl mit 1 bis 4 C-Atomen, CF₃, CH₂CF₃ oder CF₂CF₃
sowie von deren pharmazeutisch verträglichen Salzen.

Besonders bevorzugt ist die Verwendung von Verbindungen der Formel I, worin bedeuten:
- R1 und R5: unabhängig voneinander H, F, Cl, Br, CN, Methyl, Ethyl, iso-Propyl, CF₃, Cyclopropyl, OH, O-Methyl, O-Ethyl, O-iso-Propyl, OCF₃, O-Acetyl, NH₂, NMe₂, NEt₂, N-Pyrrolidino, N-Piperidino, N-Morpholino, N-(N'-Methyl)-piperazino, NHSO₂Me, Acetyl, COOH, COOR14, CONR11R12, SO₂R15 oder O-Phenyl,
R11 und R12 unabhängig voneinander H, Methyl oder Ethyl;
R14 Methyl oder Ethyl;
R15 CH₃, CF₃, OH, OCH₃, OCF₃ oder NR21 R22;
R21 und R22 unabhängig voneinander H oder Methyl; jedoch R1 und R5 nicht gleichzeitig Cl oder CH₃ sind, und worin höchstens einer der Substituenten R1 und R5 Wasserstoff ist;
- R2, R3 und R4: H;
- R6, R7, R8 und R9: unabhängig voneinander H, F, Cl, CN, CH₃, C₂H₅, Isopropyl, CF₃, Cyclopropyl, OH, OCH₃, OCF₃, O-Acetyl oder NR25R26;
R25 und R26 unabhängig voneinander H, Methyl oder Acetyl;
sowie von deren pharmazeutisch verträglichen Salzen.

Ganz besonders bevorzugt ist die Verwendung von Verbindungen der Formel I, worin bedeuten:
- R1 und R5: unabhängig voneinander F, Cl, Br, CN, Methyl, Ethyl, iso-Propyl, CF₃, Cyclopropyl, OH, O-Methyl, O-Ethyl, O-iso-Propyl, OCF₃, O-Acetyl, NH₂, NMe₂, NEt₂, N-Pyrrolidino, N-Piperidino, N-Morpholino, N-(N'-Methyl)-piperazino, NHSO₂Me, Acetyl, COOH, COOR14, CONR11 R12, SO₂R15 oder O-Phenyl,
R11 und R12 unabhängig voneinander H, Methyl oder Ethyl;
R14 Methyl oder Ethyl,
R15 CH₃, CF₃, OH, OCH₃, OCF₃ oder NR21 R22;
R21 und R22 unabhängig voneinander H oder Methyl; jedoch R1 und R5 nicht gleichzeitig Cl oder CH₃ sind, und höchstens einer der Substituenten R1 und R5 Wasserstoff bedeutet,
- R2, R3 und R4: H;
- R6 und R9: unabhängig voneinander H, F, Cl, CN, CH₃, CF₃, Cyclopropyl, OH, OCH₃, OCF₃, O-Acetyl oder NR25R26;
R25 und R26 unabhängig voneinander H, Methyl oder Acetyl;
- R7 und R8: unabhängig voneinander H, F oder OH;
sowie von deren pharmazeutisch verträglichen Salzen

Ganz besonders speziell bevorzugt ist die Verwendung von Verbindungen der Formel I, die sind:
1: (1H-Benzimidazol-2-yl)-(2,6-dichloro-phenyl)-amin;
2: 2-(2,6-Dichloro-phenylamino)-1H-benzimidazol-4-ol;
3: (1H-Benzimidazol-2-yl)-(2,6-dimethyl-phenyl)-amin;
4: (1H-Benzimidazol-2-yl)-(2-chlor-6-methyl-phenyl)-amin;
5: (2,6-Dichlor-phenyl)-(5,6-difluor-1H-Benzimidazol-2-yl)-amin;
6: (2,6-Dichlor-phenyl)-(4-methyl-1H-Benzimidazol-2-yl)-amin;
7: (1H-Benzimidazol-2-yl)-(2-chlor-6-fluor-phenyl)-amin;
8: (1H-Benzimidazol-2-yl)-(2,6-dibrom-phenyl)-amin;
9: 2-(2,6-Dichlorphenylamino)-5-fluorbenzimidazol;
10: 2-(2,6-Dichlorphenylamino)-4-fluorbenzimidazol;
11: 2-(2-Trifluormethyl-6-chlorphenylamino)benzimidazol;
12: 2-(2,6-Dichlorphenylamino)-4,5-difluorbenzimidazol;
13: 2-(2,6-Dichlorphenylamino)-5-hydroxybenzimidazol;
14: 2-(2,6-Dichlorphenylamino)-4,5,6,7-tetrafluorbenzimidazol;
15: 2-(2,6-Dichlorphenylamino)-4,6-difluorbenzimidazol;
16: (1H-Benzimidazol-2-yl)-(2-chlor-phenyl)-amin;
17: (1H-Benzimidazol-2-yl)-(2-trifluormethyl-phenyl)-amin;
18: (1H-Benzimidazol-2-yl)-(2-brom-phenyl)-amin; und
19: (1H-Benzimidazol-2-yl)-o-tolyl-amin;
und von ihren pharmazeutisch verträglichen Salzen.

Enthalten die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die Verbindungen der Formel I können weiterhin als Tautomere oder als Gemisch tautomerer Strukturen vorliegen. Bei Substitution an den entsprechenden N-Atomen der Benzimidazolstruktur können die Verbindungen in Form der verschiedenen Doppelbindungsisomere oder als Gemisch der Doppelbindungsisomere vorliegen.

Die bezeichneten Alkylreste, bzw. teilweise oder vollständig fluorierten Alkylreste können sowohl geradkettig als auch verzweigt vorliegen.

Als CH₂-Einheiten gelten auch die in einer Alkylkette terminalen CH₃-Gruppen, die in diesem Zusammenhang als CH₂-H Gruppierungen aufgefasst werden.

Beschrieben werden auch Methoden zur Herstellung der verwendeten Verbindungen. So lassen sich die durch Formel I beschriebenen Substanzen in dem Fachmann bekannter Weise aus den zugrundeliegenden Isothiocyanaten II und den entsprechenden Phenylendiaminen III herstellen.

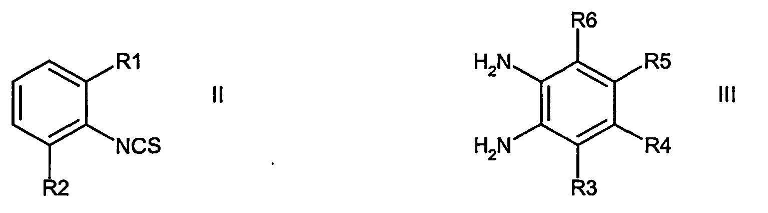

Der hierbei intermediär gebildete Thioharnstoff wird dabei mittels Quecksilber-II-oxid (J. Med. Chem., 1975, 18, 90 - 99), Methyliodid (Synthesis, 1974, 41 - 42) oder Carbodiimid (Synthesis, 1977, 864 - 865) zum entsprechenden Benzimidazol I cyclisiert. Die hierbei verwendeten Isothiocyanate II können, falls nicht käuflich erhältlich, in literaturbekannter Weise aus den entsprechenden Anilinen durch dem Fachmann bekannte Methoden, z. B. durch Behandeln mit Thiophosgen (J. Med. Chem., 1975, 18, 90-99) oder Thiocarbonyldiimidazol (Justus Liebigs Ann. Chem., 1962, 657) hergestellt werden.

Ebenfalls ausgehend aus den Anilinen lassen sich durch Behandeln mit NaOH, Schwefelkohlenstoff und Methyliodid in bereits literaturbekannten Verfahren die entsprechenden N-Aryldithiocarbamate (Synthesis, 1981, 961) und daraus wiederum die N-Aryldithiocarbonimidate IV herstellen (Synthesis, 1983, 375), die in Gegenwart der Phenylendiamine III bei erhöhten Temperaturen zu den gewünschten Benzimidazolen I umgesetzt werden können.

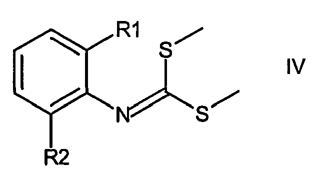

Schließlich lassen sich die beschriebenen Verbindungen I ausgehend aus den Anilinen und den entsprechendend 2-substituierten Benzimidazolen V durch Erhitzen herstellen.

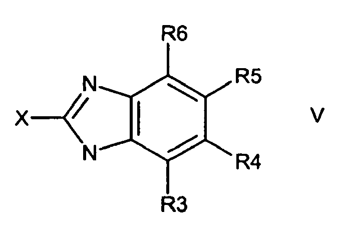

X stellt hierbei eine Abgangsgruppe, wie z. B. Cl, Br, oder SO₃H (J. Org. Chem., 1986, 51, 1882) dar.

Die englische Patentschrift 1 171 904 beschreibt eine allgemeine Formel, welche bereits o,o-Disubstitution im Anilinteil erlauben würde. Jedoch findet sich kein Hinweis auf tatsächlich in Betracht gezogene Verbindungen des Typs I, die ein o,o-Disubstitutionsmuster aufweisen, geschweige denn eine experimentelle Beschreibung. Die in dieser englischen Patentschrift 1 171 904 beschriebenen Verbindungen sind dort als antibakteriell wirkenden Substanzen geschützt. Bei den erfindungsgemäßen Verbindungen konnte anhand einer Beispielverbindung keine antibakterielle Wirkung nachgewiesen werden, so dass sich die Substanzklasse nach der GB 1 171 904 sowohl strukturell als auch in ihren pharmakologischen Eigenschaften deutlich von den erfindungsgemäßen Verbindungen unterscheidet.

Weiterhin könnten einige der erfindungsgemäßen Benzimidazole aus der WO 9808818 konstruiert werden, die dort als Phospholipaseinhibitoren beschrieben sind. Allerdings wird dort kein einziger Vertreter dieser Verbindungsklasse beschrieben, weder experimentell noch pharmakologisch.

In der Offenlegungsschrift EP0640588 werden ortho-substituierte Benzoylguanidine beschrieben, die Inhibitoren des Natrium-Wasserstoffaustauschers (NHE) sind.

Es konnte gezeigt werden, dass Verbindungen der Formel I hervorragende Inhibitoren des Natrium-Wasserstoffaustauschers (NHE) - insbesondere des Natrium-Wasserstoffaustauschers vom Subtyp 3 (NHE3) - darstellen.

Aufgrund dieser Eigenschaften eignen sich die Verbindungen zur Behandlung von Krankheiten, die durch Sauerstoffmangel hervorgerufen werden. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺⁻Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäß verwendeten Verbindungen der Formel I ebenfalls zur Behandlung von Formen des Schocks, wie beispielweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z. B. zentrale Schlafapnoen, plötzlicher Kindstod, postoperative Hypoxie), muskulärbedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Zusätzlich erhöhen die Verbindungen den Muskeltonus der oberen Atemwege, so dass das Schnarchen unterdrückt wird.

Eine Kombination eines NHE-Inhibitors mit einem Carboanhydrase-Hemmer (z. B. Acetazolamid), wobei letzterer eine metabolische Azidose herbeiführt und dadurch bereits die Atmungstätigkeit steigert, erweist sich als vorteilhaft durch verstärkte Wirkung und verminderten Wirkstoffeinsatz.

Es hat sich gezeigt, dass die erfindungsgemäß verwendeten Verbindungen eine milde abführende Wirkung besitzen und demzufolge vorteilhaft als Abführmittel oder bei drohender Darmverstopfung verwendet werden können, wobei die Verhinderung der mit Verstopfungen im Darmbereich einhergehenden ischämischen Schäden besonders vorteilhaft ist.

Weiterhin besteht die Möglichkeit, der Gallenstein-Bildung vorzubeugen.

Darüber hinaus zeichnen sich die erfindungsgemäß verwendeten Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäß verwendeten Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Es wurde außerdem gefunden, dass NHE-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Die erfindungsgemäß verwendeten Verbindungen können daher zur Prophylaxe und zur Regression von atherosklerotischen Veränderungen herangezogen werden, indem sie einen kausalen Risikofaktor ausschalten. Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens; zur Herstellung eines Medikaments zur Blutdrucksenkung; zur Herstellung eines Medikaments zur Prävention und Behandlung von Erkrankungen, die durch Ischämie und Reperfusion von zentralen und peripheren Organen ausgelöst werden wie das akute Nierenversagen, der Schlaganfall, endogene Schockzustände, Darmerkrankungen etc.; zur Herstellung eines Medikaments zur Behandlung diabetischer Spätschäden und chronischer Nierenerkrankungen, insbesondere von allen Nierenentzündungen (Nephritiden), die mit einer vermehrten Protein-/ Albuminausscheidung verbunden sind; zur Herstellung eines Medikaments zur Behandlung des Befalls durch Ektoparasiten in der Human- und Veterinärmedizin; zur Herstellung eines Medikaments zur Behandlung der genannten Leiden in Kombinationen mit blutdrucksenkenden Stoffen, bevorzugt mit Angiotensin Converting Enzyme (ACE)-Hemmern, mit Diuretika und Saluretika wie Furosemid, Hydrochlorothiazid, Pseudoaldosteronantagonisten und Aldosteron-Antagonisten; und mit Angiotensin-Rezeptorantagonisten.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Bevorzugt finden die genannten Verbindungen Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens; zur Herstellung eines Medikaments zur Blutdrucksenkung; zur Herstellung eines Medikaments zur Prävention und Behandlung von Erkrankungen, die durch lschämie und Reperfusion von zentralen und peripheren Organen ausgelöst werden wie das akute Nierenversagen, Darmerkrankungen etc.; zur Herstellung eines Medikaments zur Behandlung diabetischer Spätschäden und chronischer Nierenerkrankungen, insbesondere von allen Nierenentzündungen (Nephritiden), die mit einer vermehrten Protein-/ Albuminausscheidung verbunden sind; zur Herstellung eines Medikaments zur Behandlung des Befalls durch Ektoparasiten in der Human- und Veterinärmedizin; zur Herstellung eines Medikaments zur Behandlung der genannten Leiden in Kombinationen mit blutdrucksenkenden Stoffen, bevorzugt mit Angiotensin Converting Enzyme (ACE)-Hemmern, mit Diuretika und Saluretika wie Furosemid, Hydrochlorothiazid, Pseudoaldosteronantagonisten und Aldosteron-Antagonisten; mit Adenosinrezeptor-Modulatoren, insbesondere mit Adenosinrezeptor-Aktivatoren (A2-Agonisten), und mit Angiotensin-Rezeptorantagonisten.

Arzneimittel, die eine Verbindung I enthalten, können dabei oral, parenteral, intravenös, rektal, transdermal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Versuchsbeschreibungen und Beispiele:

### Liste der verwendeten Abkürzungen:

- EA: Ethylacetat
- Rₜ: Retentionszeit
- TFA: Trifluoressigsäure
- LCMS: Liquid Chromatography Mass Spectroscopy
- RT: Raumtemperatur
- THF: Tetrahydrofuran
- MS: Mass Spectroscopy
- Cl: Chemical lonization

### Allgemeines:

Die im Folgenden angegebenen Retentionszeiten (Rt) beziehen sich auf LCMS-Messungen mit den folgenden Parametern:
- stationäre Phase:: Merck Purospher 3µ2 x 55 mm
- mobile Phase:: 95% H₂O (0.05% TFA)→ 95% Acetonitril; 4 min; 95%
Acetonitril; 1.5 min → 5% Acetonitril; 1 min; 0.5 ml/min.

### Beispiel 1: (1H-Benzimidazol-2-yl)-(2,6-dichloro-phenyl)-amin-hydrochlorid

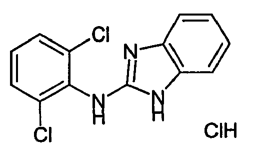

(1H-Benzimidazol-2-yl)-(2,6-dichloro-phenyl)-amin kann nach literatutbekannter Methode hergestellt werden (J. Med. Chem., 1975, 18, 90). Umkristallisation aus heißer, verdünnter Salzsäure liefert das entsprechende Hydrochlorid als farblosen Feststoff. (MS-Cl: 278.2, 280.0; LCMS-Rt = 3.605 min).

### Beispiel 2: 2-(2,6-Dichloro-phenylamino)-1H-benzimidazol-4-ol-hydrochlorid

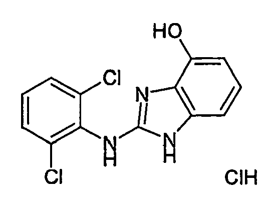

Zwischenprodukt 1: 1-(2-Amino-6-hydroxy-phenyl)-3-(2,6-dichloro-phenyl)-thioharnstoff 1,0 Äquivalente 2,6-Dichlorphenylisothiocanat werden in Essigester mit 1,0 Äquivalenten 2,3-Diaminophenol versetzt und 1 h zum Rückfluss erhitzt. Nach Abkühlen auf Raumtemperatur wird der Niederschlag abgesaugt, mit Ether gewaschen und getrocknet. Der gewünschte Thioharnstoff wird in einer Ausbeute von 61 % erhalten. (Schmp. 202-204 °C;).

### 2-(2,6-Dichloro-phenylamino)-1H-benzimidazol-4-ol-hydrochlorid

Zwischenprodukt 1 wird in Ethanol gelöst und mit 8 Äquivalenten Methyliodid versetzt. Es wird 8 h zum Rückfluss erhitzt. Nachdem auf Raumtemperatur abgekühlt wurde, wird die Reaktionslösung über Aktivkohle filtriert und das Filtrat i. Vak. eingeengt. Der Rückstand wird mit 0,5 N HCl verrieben und der Niederschlag nach 30 min abgesaugt. Der Rückstand wird noch einmal mit Essigester verrührt und getrocknet, wobei die Titelverbindung in einer Ausbeute von 47 % isoliert werden kann. (Schmp.: 333-335 °C; MS(Cl+): 294,1; 296,1).

### Beispiel 3: (1H-Benzimidazol-2-yl)-(2,6-dimethyl-phenyl)-amin-Trifluoressigsäuresalz

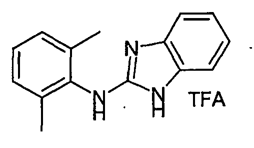

2,6-Dimethylanilin (0,5 g) und 2-Chlorbenzimidazol (0,63 g) wurden in einem Kolben vermischt und anschließend 2 h bei 200°C gehalten. Nach dem Abkühlen wurde der Rückstand mit 1 N HCl bei Siedehitze aus dem Kolben gelöst. Anschließend wurde das Herausgelöste 30 min bei RT gerührt, dann vom Unlöslichen abgesaugt und das Filtrat eingedampft. Der Rückstand wurde mittels präparativer HPLC an RP-18 mit Acetonitril/Wasser (0,05% TFA) gereinigt. Die sauberen Fraktionen wurden vereinigt, eingeengt und dann aus Acetonitril/Wasser umkristallisiert. Es wurden 500 mg weißer Kristalle erhalten.
LCMS-Rₜ: 3,30 min; MS (ES+, M+H⁺): 238,1

### Beispiel 4: (1H-Benzimidazol-2-yl)-(2-chlor-6-methyl-phenyl)-amine-Hydrochlorid

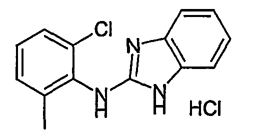

2-Chlor-6-methylanilin (0,46 g) und 2-Chlorbenzimidazol (0,5 g) wurden in einem Kolben vermischt und anschließend 30 min bei 170°C gehalten. Nach dem Abkühlen wurde der Rückstand mit 1 N HCl und 10% Ethanol bei Siedehitze aus dem Kolben gelöst. Anschließend wurde das Herausgelöste 30 min bei RT gerührt, dann vom Unlöslichen abgesaugt und das Filtrat eingedampft. Der Rückstand wurde mittels präparativer HPLC an RP-18 mit Acetonitril/Wasser (0,05% TFA) gereinigt. Die sauberen Fraktionen wurden vereinigt, das Acetonitril abgezogen, mit Kaliumcarbonat-Lösung auf pH 10 gestellt, dreimal mit EE extrahiert und anschließend die vereinigten Phasen getrocknet, filtriert und eingeengt. Der Rückstand wurde mit HCl/Wasser aufgenommen und gefriergetrocknet. Es wurden 227 mg der Titelverbindung erhalten.
LCMS-Rₜ: 3,71 min; MS (ES+, M+H⁺): 258,0

### Beispiel 5: (2,6-Dichlor-phenyl)-(5,6-difluor-1H-Benzimidazol-2-yl)-amin-Hydrochlorid

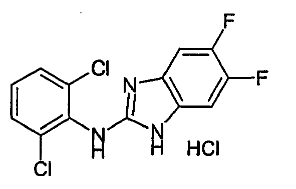

2,6-Dichlorphenyl-isothiocyanat (0,3 g) und 4,5-Difluor-1,2-phenylendiamin (0,21 g) wurden in THF (15 ml) 4 h bei RT gerührt und anschließend eingeengt und am Hochvakuum getrocknet. Der schaumige Rückstand wurde in Ethanol gelöst und unter Rühren auf 70°C erhitzt. Anschließend wurde Methyliodid (0,73 ml) zugetropft. Nach drei Stunden wurde das Heizen gestoppt und der Ansatz über Nacht stehen gelassen. Nach dem Einengen wurde mit Wasser und EE aufgenommen, die EE-Phase abgetrennt, mit Magnesiumsulfat getrocknet, filtriert und eingeengt. Der Rückstand wurde mittels präparativer HPLC an RP-18 mit Acetonitril/Wasser (0,05% TFA) gereinigt. Die sauberen Fraktionen wurden vereinigt, das Acetonitril abgezogen, mit Kaliumcarbonat-Lösung auf pH 10 gestellt, dreimal mit EE extrahiert und anschließend die vereinigten Phasen getrocknet, filtriert und eingeengt. Der Rückstand wurde mit 2 N HCl aufgenommen und gefriergetrocknet. Es wurden 55 mg der Titelverbindung erhalten.
LCMS-Rₜ: 3,83 min; MS (Cl+, M+H⁺): 314,1

### Beispiel 6: (2,6-Dichlor-phenyl)-(4-methyl-1H-Benzimidazol-2-yl)-amin-Trifluoressigsäuresalz

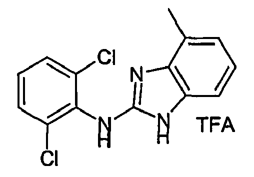

2,6-Dichlorphenyl-isothiocyanat (0,15 g) und 2,3-Diaminotoluol (0,09 g) wurden in THF (15 ml) gelöst, 4 h bei RT gerührt und anschließend mit N,N'-Dicyclohexylcarbodiimid (0,23 g) versetzt und 6 h am Rückfluss gekocht. Nach Stehenlassen über Nacht wurde das Reaktionsgemisch eingeengt, mit Acetonitril/Wasser (80:20) versetzt, das Ungelöste abfiltriert und die Lösung mittels präparativer HPLC an RP-18 mit Acetonitril/Wasser (0,05% TFA) gereinigt. Die Produkt enthaltenden Fraktionen wurden vereinigt und zur Trockne gebracht. Kristallisation aus EE/Ether/Heptan lieferte 85 mg der Titelverbindung.
LCMS-Rₜ: 3,81 min; MS (ES+, M+H⁺): 292,0

### Beispiel 7: (1H-Benzimidazol-2-yl)-(2-chlor-6-fluor-phenyl)-amin-Hydrochlorid

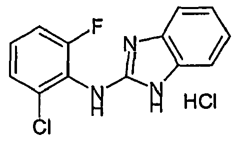

2-Chlor-6-fluoranilin (0,48 g) und 2-Chlorbenzimidazol (0,5 g) wurden in einem Kolben vermischt und anschließend 30 min bei 170°C gehalten. Nach dem Abkühlen wurde der Rückstand mit 1 N HCl und 10% Ethanol bei Siedehitze aus dem Kolben gelöst. Anschließend wurde das Herausgelöste 30 min bei RT gerührt, dann vom Unlöslichen abgesaugt und das Filtrat eingedampft. Der Rückstand wurde mittels präparativer HPLC an RP-18 mit Acetonitril/Wasser (0,05% TFA) gereinigt. Die sauberen Fraktionen wurden vereinigt, das Acetonitril abgezogen, mit Kaliumcarbonat-Lösung auf pH 10 gestellt, dreimal mit EE extrahiert und anschließend die vereinigten Phasen getrocknet, filtriert und eingeengt. Der Rückstand wurde mit HCl/Wasser aufgenommen und gefriergetrocknet. Es wurden 27 mg der Titelverbindung erhalten.
LCMS-Rₜ: 3,45 min; MS (ES+, M+H⁺): 262,0

### Beispiel 8: (1H-Benzimidazol-2-yl)-(2,6-dibrom-phenyl)-amin-Trifluoressigsäuresalz

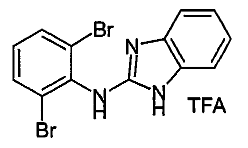

2,6-Dibromanilin (0,5 g) wurde in absolutem Dioxan (5 ml) gelöst, durch ein Septum Trimethylsilylchlorid (0,22 g) zugetropft und dann 2 h bei RT gerührt. Anschließend wurde in Dioxan gelöstes 2-Chlorbenzimidazol (0,3 g) zugegeben und am Rückfluss gekocht. Nach 4 h wurde abgekühlt, das Dioxan abgezogen und der Rückstand 10 min auf 190°C erhitzt. Nach dem Abkühlen wurde der Rückstand mit 1 N HCl bei Siedehitze aus dem Kolben gelöst. Anschließend wurde vom Unlöslichen abfiltriert und das Filtrat eingedampft. Der Rückstand wurde mittels präparativer HPLC an RP-18 mit Acetonitril/Wasser (0,05% TFA) gereinigt. Die sauberen Fraktionen wurden vereinigt und gefriergetrocknet. Es wurden 2,4 mg der Titelverbindung erhalten.
LCMS-Rₜ: 3,74 min; MS (ES+, M+H⁺): 369,2

### Beispiel 9: 2-(2,6-Dichlorphenylamino)-5-fluorbenzimidazol Hydrochlorid

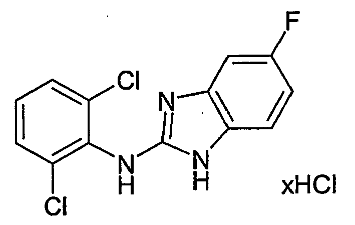

### a) 1-(2-Amino-5-fluorphenyl)-3-(2,6-dichlorphenyl)-thioharnstoff

Eine Mischung aus 4.37g (0,0346 Mol) 4-Fluor-1,2-diaminobenzol und 7.07g (0,0346 Mol) 2,6-Dichlorphenylisothiocyanat in 150 ml Ethylacetat wird 3 Std. unter Rückfluss gekocht. Nach Abdestillieren des Lösungsmittels löst man den Rückstand in Methanol, behandelt mit Aktivkohle, destilliert 2/3 des Lösungsvolumens ab und lässt den Thioharnstoff über mehrere Stunden im Eisbad kristallisieren, wobei 8.9 g des gewünschten Produkts erhalten werden. Farblose Kristalle, 1. Schmp. 175-178 °C; 2. Schmp. 294-296 °C. MS (ES+, M+ H⁺): 329.9

### b) 2-(2,6-Dichlorphenylamino)-5-fluorbenzimidazol Hydrochlorid

1-(2-Amino-5-fluorphenyl)-3-(2,6-dichlorphenyl)-thioharnstoff wird in Ethanol gelöst und mit 8 Äquivalenten Methyliodid versetzt. Es wird 6 h zum Rückfluss erhitzt. Das Lösungsmittel wird abdestilliert, der Rückstand mit Wasser versetzt, anschließend durch Zugabe von gesättigter wässriger Natriumhydrogencarbonat Lösung schwach alkalisch gestellt und extrahiert. Nach dem Abdestillieren des Lösungsmittels reinigt man durch Säulenchromatographie an Kieselgel mit einer Mischung aus Methylenchlorid und Methanol (10:1). Nach dem Abdestillieren des Lösungsmittels unter vermindertem Druck wird mit Ethylacetat gelöst, und die Lösung mit überschüssiger etherischer Salzsäure versetzt. Man rührt etwa 30 Minuten bei Raumtemperatur, filtriert die kristalline Substanz ab und kristallisiert aus einem Gemisch von Ethylacetat und Ethanol um. Farbloses kristallines Produkt, Schmp.: 294-296 °C; MS: (Cl+, M+ H+), 296

### Beispiel 10: 2-(2,6-Dichlorphenylamino)-4-fluorbenzimidazol Hydrochlorid

### a) 3-Fluor-2-nitro-phenylhydrazin

Eine Mischung aus 0,01 M 2,6-Difluornitrobenzol und 0,01 M Hydrazinhydrat (99%ig) in 30 ml Tetrahydrofuran wird über Nacht bei Raumtemperatur gerührt (exotherme Reaktion) und der Rückstand nach Abdestillieren des Lösungsmittels durch Behandeln mit Diisopropylether zur Kristallisation gebracht. Kristalline Substanz, Schmp. 93-95°C. MS (Cl+, M+ H⁺), 172.1

### b) 2,3-Diamino-fluorbenzol

erhält man durch Hydrierung von 0,0038 Mol 3-Fluor-2-nitro-phenylhydrazin in 50 ml Methanol mit Palladium auf Kohle (10%ig) als Katalysator bis zum Ende der Wasserstoffaufnahme. Nach Filtration erhält man 2,3-Diamino-fluorbenzol als gelbe ölige Substanz.
MS (Cl+, M+ H⁺),127.2

### c) 1-(2-Amino-6-fluorphenyl)-3-(2,6-dichlorphenyl)-thioharnstoff

erhält man durch Umsetzung von 0,011 M 2,3-Diamino-fluorbenzol mit 0,011 M 2,6-Dichlorphenylisothiocyanat in 30 ml wasserfreiem THF bei Raumtemperatur. Nach Abdestillieren des Lösungsmittels bringt man den Thioharnstoff unter Ethylacetat zur Kristallisation. Kristalliner Feststoff. Schmp. 315°C.
MS (Cl+, M+ H⁺),330.1

### d) 2-(2,6-Dichlorphenylamino)-4-fluorbenzimidazol Hydrochlorid

erhält man analog der in Beispiel 9 beschriebenen Vorschrift durch Umsetzung von 1-(2-Amino-6-fluorphenyl)-3-(2,6-dichlorphenyl)-thioharnstoff mit 8 Äquivalenten Methyliodid in Ethanol. Farbloser kristalliner Feststoff mit breitem Schmelzpunktsbereich von 268-296°C unter Aufschäumen.
MS (Cl+, M+ H⁺): 296.1

### Beispiel 11: 2-(2-Trifluormethyl-6-chlorphenylamino)benzimidazol Hydrochlorid

### a) 1-(2-Aminophenyl)-3-(6-chlor-2-trifluormethyl phenyl)-harnstoff

erhält man durch Umsetzung äquivalenter Mengen an 1,2-Diaminobenzol und 2-Trifluormethyl-6-chlorphenylisocyanat in wasserfreiem THF, wobei das gewünschte Harnstoffderivat nach kurzer Zeit auskristallisiert. Man rührt etwa 20 Stunden bei Raumtemperatur und filtriert den kristallinen Niederschlag ab. Zersetzungspunkt 310°C
MS (E+, M+ H⁺): 330.1

### b) 2-(2-Trifluormethyl-6-chlorphenylamino)benzimidazol Hydrochlorid

0,8g 1-(2-Aminophenyl)-3-(6-chlor-2-trifluormethyl phenyl)-harnstoff werden in 10 ml POCl₃ für 5 Stunden unter Rückflussbedingungen erhitzt, wobei eine klare Lösung entsteht. Nach dem Abdestillieren des Phosphoroxidchlorid unter vermindertem Druck behandelt man den öligen Rückstand mit Wasser, wobei langsame Kristallisation erfolgt. Man filtriert die Kristalle ab und chromatographiert an Kieselgel mit einer Mischung von 10 Teilen Dichlormethan und 1 Teil Methanol. Nach Abdestillieren des Lösungsmittels löst man den Rückstand in Ethylacetat und stellt mit einer gesättigten Lösung von Chlorwasserstoffgas in Diethylether stark sauer. Der kristalline Niederschlag wird abfiltriert. Farblose bis schwach gelbliche Kristalle. Schmp 255-288°C. MS (Cl+, M+ H⁺): 312.2

### Beispiel 12: 2-(2,6-Dichlorphenylamino)-4,5-difluorbenzimidazol Hydrochlorid S0100308, 32902-001-15834

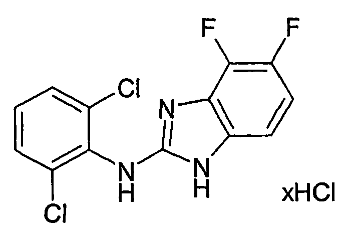

### a) 1-(2-Amino-5,6-difluorphenyl)-3-(2,6-dichlorphenyl)-thioharnstoff

erhält man durch Kochen einer Mischung von 0,01 M 1,2-Diamino-3,4-difluorbenzol mit 0,01 M 2,6-Dichlorphenylisothiocyanat in 50 ml Ethylacetat über 4 Stunden. Nach Abdestillieren des Lösungsmittels bringt man den Thioharnstoff unter Diisopropylether zur Kristallisation. Kristalliner Feststoff. Schmp. >310°C.
MS (Cl+, M+ H⁺): 348.0

### b) 2-(2,6-Dichlorphenylamino)-4,5-difluorbenzimidazol Hydrochlorid

erhält man analog der in Beispiel 9.b) beschrieben Vorschrift aus 3,2 g 1-(2-Amino-5,6-difluorphenyl)-3-(2,6-dichlorphenyl)-thioharnstoff und 10,6g Methyliodid. Kristalliner Feststoff, Schmp 228-230°C. MS (Cl+, M+ H⁺): 314.0

### Beispiel 13: 2-(2,6-Dichlorphenylamino)-5-hydroxybenzimidazol Hydrobromid

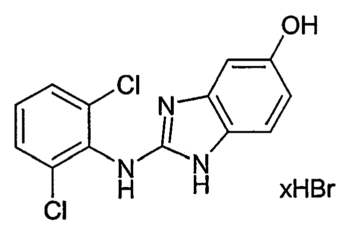

### a) 1-(2-Amino-5-methoxyphenyl)-3-(2,6-dichlorphenyl)thioharnstoff

erhält man analog der in Beispiel 12a) beschriebenen Vorschrift aus 0,005 M 1,2-Diamino-4-methoxybenzol und 0,005 M 2,6-Dichlorphenylisothiocyanat. Kristalliner Feststoff. Schmp.: 164-166°C und erneuter Kristallisation; Zersetzungspunkt: 200°C.
MS (ES+, M+): 342.0

### b) 2-(2,6-Dichlorphenylamino)-5-methoxybenzimidazol Hydrochlorid

erhält man analog der in Beispiel 9 beschriebenen Vorschrift durch Umsetzung von 0,0025 M 1-(2-Amino-5-methoxyphenyl)-3-(2,6-dichlorphenyl)-thioharnstoff mit 0,0205 M Methyliodid in 20 ml Ethanol. Nach Abdestillieren des Lösungsmittels löst man den Rückstand in wenig Ethylacetat, stellt mit einer gesättigten Lösung aus Chlorwasserstoffgas in Diethylether stark sauer und filtriert nach einigen Stunden die Kristalle ab. Schmp. 172-174°C , MS (Cl+, M+ H⁺): 308.0

### c) 2-(2,6-Dichlorphenylamino)-5-hydroxybenzimidazol Hydrobromid

Eine Mischung aus 0,05g 2-(2,6-Dichlorphenylamino)-5-methoxybenzimidazol Hydrochlorid, 0,5 ml Eisessig und 0,5 ml Bromwasserstoffsäure (48%ig) werden 3 Stunden am Rückfluss gekocht und anschließend das Lösungsmittel abdestilliert. Der feste Rückstand wird unter wenig Ethylacetat zur Kristallisation gebracht. Man erhält 0,02g 2-(2,6-Dichlorphenylamino)-5-hydroxybenzimidazol Hydrobromid vom Schmp. 265-269°C, MS (Cl+, M+ H⁺): 294.1

### Beispiel 14: 2-(2,6-Dichlorphenylamino)-4,5,6,7-tetrafluorbenzimidazol Hydrochlorid

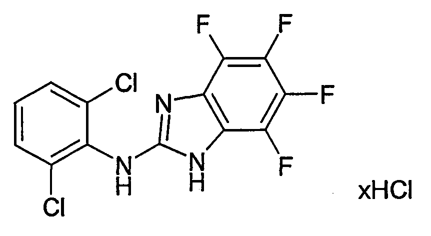

### a) 1-(2-Amino-3,4,5,6-tetrafluorphenyl)-3-(2,6-dichlorphenyl)-thioharnstoff

erhält man durch Kochen einer Mischung von 1 g 1,2-Diamino-3,4,5,6-tetrafluorbenzol mit 1,13 g 2,6-Dichlorphenylisothiocyanat in 30 ml wasserfreiem Tetrahydrofuran über 4 Stunden. Nach Abdestillieren des Lösungsmittels bringt man den Thioharnstoff unter Diisopropylether zur Kristallisation und erhält 1,88g 1-(2-Atrino-3,4,5,6-tetrafluorphenyl)-3-(2,6-dichlorphenyl)-thioharnstoff als Kristallinen Feststoff. Schmp. >300°C. MS (ES+, M+ H⁺): 384.06

### b) 2-(2,6-Dichlorphenylamino)-4,5,6,7-tetrafluorbenzimidazol Hydrochlorid

erhält man analog der in Beispiel 9.b) beschrieben Vorschrift aus 1,5 g 1-(2-Amino-3,4,5,6-tetrafluorphenyl)-3-(2,6-dichlorphenyl)-thioharnstoff und 4,4g Methyljodid und anschließende Säulenchromatographie an Kieselgel mit einem Gemisch aus 10 Teilen Ethylacetat, 5 Teilen n-Heptan, 5 Teilen Dichlormethan, 5 Teilen Methanol und 1 Teil wässrigen konzentrierten Ammoniak. Kristalliner Feststoff, Schmp 220-222°C.
MS (Cl+, M+ H⁺): 350.2

### Beispiel 15: 2-(2,6-Dichlorphenylamino)-4,6-difluorbenzimidazol Hydrochlorid

### a) 1,2-Diamino-3,5-difluorbenzol

erhält man durch Hydrierung von 5g 2-Amino-3,5-difluor-nitrobenzol mit 0,8g Pallladiumkatalysator auf Kohle bei Raumtemperatur und Normaldruck. Nach Abdestillieren des Lösungsmittels erhält man ein dunkles teilweise kristallines Öl, das ohne weitere Reinigung zur Herstellung von Stufe b) verwendet wird.

### b) 1-(2-Amino-4,6-difluorphenyl)-3-(2,6-dichlorphenyl)-thioharnstoff

erhält man durch Stehenlassen einer Mischung von 0,01 M 1,2-Diamino-3,5-difluorbenzol mit 0,01 M 2,6-Dichlorphenylisothiocyanat in 60 ml wasserfreiem THF über das Wochenende bei Raumtemperatur. Nach Abdestillieren des Lösungsmittels bringt man den Thioharnstoff unter Diisopropylether zur Kristallisation. Kristalliner Feststoff. Schmp. 310-314°C. MS (Cl+, M+ H⁺): 348.1

### c) 2-(2,6-Dichlorphenylamino)-4,5-difluorbenzimidazol Hydrochlorid

erhält man analog der in Beispiel 9.b) beschrieben Vorschrift aus 2 g 1-(2-Amino-4,6-difluorphenyl)-3-(2,6-dichlorphenyl)-thioharnstoff und 6,4 g Methyljodid. Kristalliner Feststoff, Schmp 232-234°C. MS (Cl+, M+ H⁺): 314.2

### Beispiel 16: (1H-Benzimidazol-2-yl)-(2-chlor-phenyl)-amin-Trifluoressigsäuresalz

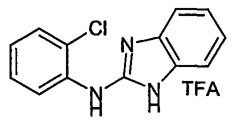

2-Chloranilin (0,5 g) und 2-Chlorbenzimidazol (0,6 g) wurden in einem Kolben vermischt und anschließend 2 h bei 225°C gehalten. Nach dem Abkühlen wurde der Rückstand mit 1 N HCl bei Siedehitze aus dem Kolben gelöst, vom Unlöslichen abgesaugt, das Filtrat mit Kaliumcarbonat auf pH 9-10 gestellt und eingeengt. Der Rückstand wurde mit heißem Methanol behandelt, das Ungelöste abfiltriert, die Mutterlauge mit Ether versetzt und der Niederschlag erneut abfiltriert. Die Mutterlauge wurde eingeengt und der Rückstand erneut aus Methanol/Ether kristallisiert. Nach Absaugen der Kristalle wurde die Mutterlauge eingeengt und der Rückstand mittels präparativer HPLC an RP-18 mit Acetonitril/Wasser (0,05% TFA) gereinigt. Die sauberen Fraktionen wurden vereinigt und gefriergetrocknet. Es wurden 100 mg der Titelverbindung erhalten.
LCMS-Rₜ: 3,16 min; MS (Cl+, M+H⁺): 244,0

### Beispiel 17: (1H-Benzimidazol-2-yl)-(2-trifluormethyl-phenyl)-amin-Trifluoressigsäuresalz

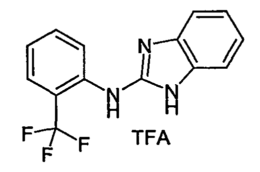

2-Aminobenzotrifluorid (0,5 g) und 2-Chlorbenzimidazol (0,47 g) wurden in einem Kolben vermischt und anschließend 2 h bei 225°C gehalten. Nach dem Abkühlen wurde der Rückstand mit 1 N HCl bei Siedehitze aus dem Kolben gelöst und nach Erkalten vom Unlöslichen abgesaugt. Das Filtrat wurde eingeengt und der Rückstand mittels präparativer HPLC an RP-18 mit Acetonitril/Wasser (0,05% TFA) gereinigt. Die sauberen Fraktionen wurden vereinigt und gefriergetrocknet. Es wurden 52 mg der Titelverbindung erhalten.
LCMS-Rₜ: 3,65 min; MS (Cl+, M+H⁺): 278,1

### Beispiel 18: (1H-Benzimidazol-2-yl)-(2-brom-phenyl)-amin-Trifluoressigsäuresalz

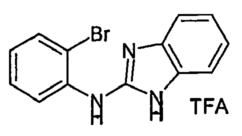

### 2-Bromanilin (0,5 g) und 2-Chlorbenzimidazol (0,44 g) wurden entsprechend Beispiel 17 umgesetzt.

Es wurden 117 mg der Titelverbindung erhalten.
LCMS-Rₜ: 3,55 min; MS (ES+, M+H⁺): 288,0

### Beispiel 19: (1H-Benzimidazol-2-yl)-o-tolyl-amin-Hydrochlorid

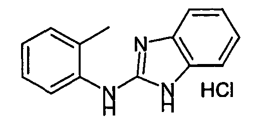

2-Methylanilin (0,5 g) und 2-Chlorbenzimidazol (0,71 g) wurden in einem Kolben vermischt und anschließend 2 h bei 250°C gehalten. Nach dem Abkühlen wurde der Rückstand mit 1 N HCl bei Siedehitze aus dem Kolben gelöst, dann vom Unlöslichen abgesaugt und das Filtrat eingedampft. Der Rückstand wurde mittels präparativer HPLC an RP-18 mit Acetonitril/Wasser (0,05% TFA) gereinigt. Die sauberen Fraktionen wurden vereinigt, das Acetonitril abgezogen, mit Kaliumcarbonat-Lösung alkalisch gestellt, dreimal mit EE extrahiert und anschließend die vereinigten Phasen getrocknet, filtriert und eingeengt. Der Rückstand wurde mit 2 N HCl aufgenommen und gefriergetrocknet. Es wurden 110 mg der Titelverbindung erhalten.
LCMS-Rₜ: 3,54 min; MS (Cl+, M+H⁺): 224,1

### Pharmakologische Daten:

### Testbeschreibung:

in diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl-Puffer: 115 mM NaCl, 20 mM NH₄Cl, 5 mM KCl, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl - Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCl, 1,25 mM CaCl₂, 1,25 mM MgCl₂, 0,97 mM Na₂HPO₄, 0,23 mM Na₂HPO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCl, 1,25 mM CaCl₂, 1,25 mM MgCl₂, 0,97 mM K₂HPO₄, 0,23 mM KH₂PO₄, 5 mM Hepes, 5 mM Glucose, mit 1 M NaOH wird ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC-Wert der jeweiligen Substanz für die einzelnen NHE-Subtypen berechnet.

### Ergebnisse:

| Beispiel | IC₅₀ [µM], (rNHE3) |
|---|---|
| 1 | 0.53 |
| 2 | 0,47 |
| 3 | 0.64 |
| 4 | 0,49 |
| 5 | 0,78 |
| 6 | 0,39 |
| 7 | 0,52 |
| 8 | 0,65 |
| 9 | 1,0 |
| 10 | 3,2 |
| 11 | 0,83 |
| 12 | 2,9 |
| 13 | 1,1 |
| 14 | 5,6 |
| 15 | 1,6 |
| 16 | 0,63 |
| 17 | 3,5 |
| 18 | 1,2 |
| 19 | 3,5 |

## Patentansprüche

1. Verwendung von Verbindungen der Formel I 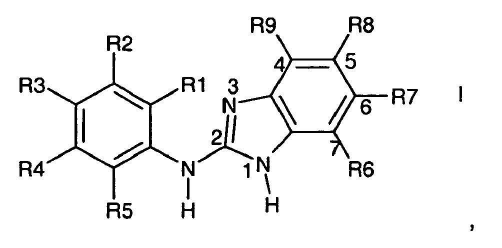 worin bedeuten:
R1 und R5 unabhängig voneinander H, F, Cl, Br, I, CN, Alkyl mit 1 bis 4 C-Atomen,
welches unsubstituiert ist oder teilweise oder vollständig mit Fluor substituiert ist,
oder
R1 und R5 Cycloalkyl mit 3 bis 7 C-Atomen,
welches unsubstituiert ist oder teilweise oder vollständig mit Fluor substituiert ist,
oder
R1 und R5 OH, O-Alkyl mit 1 bis 4 C-Atomen,
welches unsubstituiert ist oder teilweise oder vollständig mit Fluor substituiert ist,
oder
R1 und R5 OCOR10, NR11R12, COR13, COOH, COOR14, CONR11R12, -(O)n SOₘR15,
n Null oder 1;
m Null, 1 oder 2;
oder
R1 und R5
O-Phenyl, welches unsubstituiert ist oder substituiert mit 1 - 3 Substituenten ausgewählt aus der Gruppe bestehend aus F, Cl, Br, J, Alkyl mit 1 bis 4 C-Atomen, OH, O-Alkyl mit 1 bis 4 C-Atomen, NR16R17, CN oder (C₁-C₄)- Alkylsulfonyl,
welches unsubstituiert ist oder teilweise oder ganz fluoriert,
R16 und R17 H oder Alkyl mit 1 bis 4 C-Atomen,
wobei die Alkylgruppen unsubstituiert sind oder teilweise oder ganz fluoriert,
R10 H oder Alkyl mit 1 bis 4 C-Atomen,
welches unsubstituiert ist oder teilweise oder vollständig mit Fluor substituiert ist,
R11 und R12 unabhängig voneinander H, Alkyl mit 1 bis 4 C-Atomen, die teilweise oder vollständig fluoriert sein dürfen und eine beliebige CH₂-Gruppe durch O oder NR18 ersetzt sein darf
oder R11 und R12 gemeinsam einen 5-, 6- oder 7-gliedrigen Ring;
oder R11 und R12 COR19 oder SO₂R20;
R18, R19 und R20 unabhängig voneinander H oder Alkyl mit 1 bis 4 C-Atomen,
welches unsubstituiert ist oder teilweise oder vollständig mit Fluor substituiert ist;
R13 und R14 Alkyl mit 1 bis 4 C-Atomen,
welches unsubstituiert ist oder teilweise oder vollständig mit Fluor substituiert ist;
R15 Alkyl oder O-Alkyl mit 1 bis 4 C-Atomen,
wobei die Alkylgruppen unsubstituiert sind oder teilweise oder ganz fluoriert,
oder
R15 OH oder NR21 R22;
R21 und R22 unabhängig voneinander H oder Alkyl mit 1 bis 4 C-Atomen, welches unsubstituiert ist oder teilweise oder vollständig mit Fluor substituiert ist, worin eine beliebige CH₂-Gruppe durch O oder NR23 ersetzt sein kann;
R23 H oder Alkyl mit 1 bis 4 C-Atomen,
welches unsubstituiert ist oder teilweise oder vollständig mit Fluor substituiert ist;
oder
R21 und R22 gemeinsam einen 5-, 6- oder 7-gliedrigen Ring bilden;
R2, R3 und R4 H oder F;
R6, R7, R8 und R9 unabhängig voneinander H, F, Cl, Br, I, CN, Alkyl oder O-Alkyl mit 1 bis 4 C-Atomen,
die unsubstituiert oder ganz oder teilweise durch Fluor substituiert sind,
oder R6, R7, R8 und R9 Cycloalkyl mit 3 bis 7 C-Atomen,
das unsubstituiert oder ganz oder teilweise durch Fluor substituiert ist,
oder R6, R7, R8 und R9 OH, OCOR24 oder NR25R26;
R24 H oder Alkyl mit 1 bis 4 C-Atomen,
das unsubstituiert oder ganz oder teilweise durch Fluor substituiert ist;
R25 und R26 unabhängig voneinander H oder Alkyl mit 1 bis 4 C-Atomen,
das unsubstituiert oder ganz oder teilweise durch Fluor substituiert ist,
oder
R25 und R26 COR27;
oderR25 und R26 gemeinsam mit dem Stickstoffatom, an das sie gebunden sind, einen 5-, 6- oder 7-gliedrigen Ring bilden, in welchem eine beliebige CH₂-Gruppe durch O oder NR18 ersetzt sein kann;
R27 H oder Alkyl mit 1 bis 4 C-Atomen, das unsubstituiert oder ganz oder teilweise durch Fluor substituiert ist,
sowie von deren pharmazeutisch verträglichen Salzen,
zur Herstellung eines Medikaments zur Behandlung oder Prophylaxe von Störungen des Atemantriebs, von Atemstörungen, insbesondere Schlaf-bedingten Atemstörungen wie Schlafapnoen, des Schnarchens, von akuten und chronischen Nierenerkrankungen, besonders des akuten Nierenversagens und des chronischen Nierenversagens, von Störungen der Darmfunktion, von Störungen der Gallenfunktion, von ischämischen Zuständen des peripheren und zentralen Nervensystems und des Schlaganfalls, von ischämischen Zuständen peripherer Organe und Gliedmaßen, von Schockzuständen, von Krankheiten, bei denen die Zellproliferation eine primäre oder sekundäre Ursache darstellt, von Störungen des Fettstoffwechsels, des Befalls durch Ektoparasiten in der Human- und Veterinärmedizin zur Herstellung eines Medikaments zum Einsatz bei chirurgischen Operationen und Organtransplantationen und zur Herstellung eines Medikaments zur Konservierung und Lagerung von Transplantaten für chirurgische Maßnahmen.

2. Verwendung einer Verbindung I nach Anspruch 1 zur Herstellung eines Medikaments zur Behandlung oder Prophylaxe von Störungen des Atemantriebs.

3. Verwendung einer Verbindung I nach Anspruch 1 zur Herstellung eines Medikaments zur Behandlung oder Prophylaxe von Atemstörungen, insbesondere Schlaf-bedingten Atemstörungen wie Schlafapnoen.

4. Verwendung einer Verbindung I nach Anspruch 1 zur Herstellung eines Medikaments zur Behandlung oder Prophylaxe des Schnarchens.

5. Verwendung einer Verbindung I nach Anspruch 1 zur Herstellung eines Medikaments zur Behandlung oder zur Prophylaxe von akuten und chronischen Nierenerkrankungen, besonders des akuten Nierenversagens und des chronischen Nierenversagens.

6. Verwendung einer Verbindung I Anspruch 1 zur Herstellung eines Medikaments zur Behandlung oder Prophylaxe von Störungen der Darmfunktion.

7. Verwendung einer Verbindung I nach Anspruch 1 zur Herstellung eines Medikaments zur Behandlung oder Prophylaxe von Störungen der Gallenfunktion.

8. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

9. Verwendung einer Verbindung I nach Anspruch 1 zur Herstellung eines Medikaments zur Behandlung oder Prophylaxe von ischämischen Zuständen peripherer Organe und Gliedmaßen.

10. Verwendung einer Verbindung I nach Anspruch 1 zur Herstellung eines Medikaments zur Behandlung von Schockzuständen.

11. Verwendung einer Verbindung I nach Anspruch 1 zur Herstellung eines Medikaments zu Einsatz bei chirurgischen Operationen und Organtransplantationen.

12. Verwendung einer Verbindung I nach Anspruch 1 zur Herstellung eines Medikaments zur Konservierung und Lagerung von Transplantaten für chirurgische Maßnahmen.

13. Verwendung einer Verbindung I nach Anspruch 1 zur Herstellung eines Medikaments zur Behandlung von Krankheiten, bei denen die Zellproliferation eine primäre oder sekundäre Ursache darstellt.

14. Verwendung einer Verbindung I nach Anspruch 1 zur Herstellung eines Medikaments zur Behandlung oder Prophylaxe von Störungen des Fettstoffwechsels.

15. Verwendung einer Verbindung I nach Anspruch 1 zur Herstellung eines Medikaments zur Behandlung des Befalls durch Ektoparasiten in der Human- und Veterinärmedizin.

16. Verbindung der Formel I 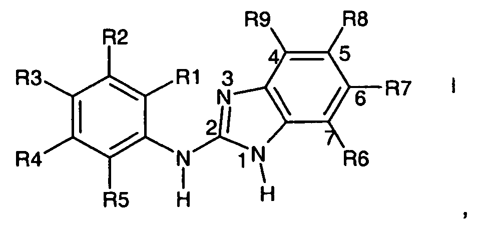 die ist
2-(2,6-Dichloro-phenylamino)-1H-benzimidazol-4-ol;
(1H-Benzimidazol-2-yl)-(2-chlor-6-methyl-phenyl)-amin;
(2,6-Dichlor-phenyl)-(5,6-difluor-1H-Benzimidazol-2-yl)-amin;
(2,6-Dichlor-phenyl)-(4-methyl-1H-Benzimidazol-2-yl)-amin;
(1H-Benzimidazol-2-yl)-(2-chlor-6-fluor-phenyl)-amin;
(1H-Benzimidazol-2-yl)-(2,6-dibrom-phenyl)-amin;
2-(2,6-Dichlorphenylamino)-5-fluorbenzimidazol;
2-(2,6-Dichlorphenylamino)-4-fluorbenzimidazol;
2-(2-Trifluormethyl-6-chlorphenylamino)benzimidazol;
2-(2,6-Dichlorphenylamino)-4,5-difluorbenzimidazol;
2-(2,6-Dichlorphenylamino)-5-hydroxybenzimidazol;
2-(2,6-Dichlorphenylamino)-4,5,6,7-tetrafluorbenzimidazol;
2-(2,6-Dichlorphenylamino)-4,6-difluorbenzimidazol;
(1H-Benzimidazol-2-yl)-(2-trifluormethyl-phenyl)-amin;
oder
(1H-Benzimidazol-2-yl)-(2-brom-phenyl)-amin;
und ihre pharmazeutisch verträglichen Salze oder Trifluoracetate.

17. Medikament enthaltend eine Verbindung der Formel I nach Anspruch 16.

## Claims

1. The use of compounds of the formula I 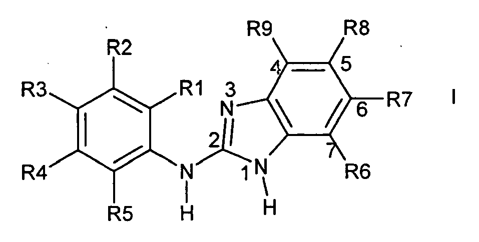 in which:
R1 and R5 independently of one another are H, F, Cl, Br, I, CN, alkyl having 1 to 4 carbon atoms,
which is unsubstituted or partially or completely substituted by fluorine,
or
R1 and R5 are cycloalkyl having 3 to 7 carbon atoms,
which is unsubstituted or partially or completely substituted by fluorine,
or
R1 and R5 are OH, O-alkyl having 1 to 4 carbon atoms,
which is unsubstituted or partially or completely substituted by fluorine,
or
R1 and R5 are OCOR10, NR11R12, COR13, COOH, COOR14, CONR11R12, -(O)n SOₘR15,
n is zero or 1;
m is zero, 1 or 2;
or
R1 and R5 are
O-phenyl, which is unsubstituted or substituted by 1 - 3 substituents selected from the group consisting of F, Cl, Br, I, alkyl having 1 to 4 carbon atoms, OH, O-alkyl having 1 to 4 carbon atoms, NR16R17, CN or (C₁-C₄)-alkylsulfonyl,
which is unsubstituted or partially or wholly fluorinated,
R16 and R17 are H or alkyl having 1 to 4 carbon atoms,
where the alkyl groups are unsubstituted or partially or wholly fluorinated,
R10 is H or alkyl having 1 to 4 carbon atoms,
which is unsubstituted or partially or completely substituted by fluorine,
R11 and R12 independently of one another are H, alkyl having 1 to 4 carbon atoms, which may be partially or completely fluorinated and any desired CH₂ group may replaced be by O or NR18
or R11 and R12 together are a 5-, 6- or 7-membered ring;
or R11 and R12 are COR19 or SO₂R20;
R18, R19 and R20 independently of one another are H or alkyl having 1 to 4 carbon atoms,
which is unsubstituted or partially or completely substituted by fluorine;
R13 and R14 are alkyl having 1 to 4 carbon atoms,
which is unsubstituted or partially or completely substituted by fluorine;
R15 is alkyl or O-alkyl having 1 to 4 carbon atoms,
where the alkyl groups are unsubstituted or partially or wholly fluorinated,
or
R15 is OH or NR21 R22;
R21 and R22 independently of one another are H or alkyl having 1 to 4 carbon atoms,
which is unsubstituted or partially or completely substituted by fluorine, in which any desired CH₂ group can be replaced by O- or NR23;
R23 is H or alkyl having 1 to 4 carbon atoms,
which is unsubstituted or partially or completely substituted by fluorine;
or
R21 and R22 together form a 5-, 6- or 7-membered ring;
R2, R3 and R4 are H or F;
R6, R7, R8 and R9 independently of one another are H, F, Cl, Br, I, CN, alkyl or O-alkyl having 1 to 4 carbon atoms,
which are unsubstituted or wholly or completely substituted by fluorine,
or R6, R7, R8 and R9 are cycloalkyl having 3 to 7 carbon atoms,
which is unsubstituted or wholly or completely substituted by fluorine,
or R6, R7, R8 and R9 are OH, OCOR24 or NR25R26;
R24 is H or alkyl having 1 to 4 carbon atoms,
which is unsubstituted or wholly or completely substituted by fluorine;
R25 and R26 independently of one another are H or alkyl having 1 to 4 carbon atoms,
which is unsubstituted or wholly or completely substituted by fluorine,
or
R25 and R26 are COR27;
or R25 and R26 together with the nitrogen atom to which they are bonded, form a 5-, 6- or 7-membered ring, in which any desired CH₂ group can be replaced by O or NR18;
R27 is H or alkyl having 1 to 4 carbon atoms,
which is unsubstituted or wholly or completely substituted by fluorine,
or of their pharmaceutically tolerable salts,
in the manufacture of a medicament for the treatment or prophylaxis of disorders of the respiratory drive, of respiratory disorders, in particular sleep-related respiratory disorders such as sleep apneas, of snoring, of acute and chronic kidney diseases, particularly acute kidney failure and chronic kidney failure, of disorders of intestinal function, of disorders of bile function, of ischemic conditions of the peripheral and central nervous system and of stroke, of ischemic conditions of peripheral organs and limbs, of states of shock, of illnesses in which cell proliferation is a primary or secondary cause, of disorders of lipid metabolism, of attack by ectoparasites in human and veterinary medicine, in the manufacture of a medicament for use in surgical operations and organ transplantations and in the manufacture of a medicament for the preservation and storage of transplants for surgical measures.

2. The use of a compound I as claimed in claim 1 in the manufacture of a medicament for the treatment or prophylaxis of disorders of the respiratory drive.

3. The use of a compound I as claimed in claim 1 in the manufacture of a medicament for the treatment or prophylaxis of respiratory disorders, in particular sleep-related respiratory disorders such as sleep apneas.

4. The use of a compound I as claimed in claim 1 in the manufacture of a medicament for the treatment or prophylaxis of snoring.

5. The use of a compound I as claimed in claim 1 in the manufacture of a medicament for the treatment or prophylaxis of acute and chronic kidney diseases, particularly acute kidney failure and chronic kidney failure.

6. The use of a compound I as claimed in claim 1 in the manufacture of a medicament for the treatment or prophylaxis of disorders of intestinal function.

7. The use of a compound I as claimed in claim 1 in the manufacture of a medicament for the treatment or prophylaxis of disorders of bile function.

8. The use of a compound I as claimed in claim 1 in the manufacture of a medicament for the treatment or prophylaxis of ischemic conditions of the peripheral and central nervous system and of stroke.

9. The use of a compound I as claimed in claim 1 in the manufacture of a medicament for the treatment or prophylaxis of ischemic conditions of peripheral organs and limbs.

10. The use of a compound I as claimed in claim 1 in the manufacture of a medicament for the treatment of states of shock.

11. The use of a compound I as claimed in claim 1 in the manufacture of a medicament for use in surgical operations and organ transplantations.

12. The use of a compound I as claimed in claim 1 in the manufacture of a medicament for the preservation and storage of transplants for surgical measures.

13. The use of a compound I as claimed in claim 1 in the manufacture of a medicament for the treatment of illnesses in which cell proliferation is a primary or secondary cause.

14. The use of a compound I as claimed in claim 1 in the manufacture of a medicament for the treatment or prophylaxis of disorders of lipid metabolism.

15. The use of a compound I as claimed in claim 1 in the manufacture of a medicament for the treatment of attack by ectoparasites in human and veterinary medicine.

16. A compound of the formula I 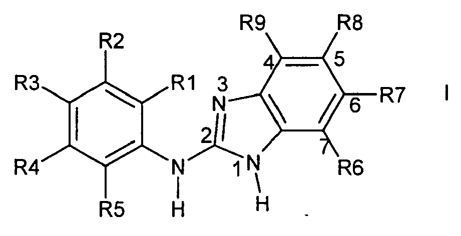 which is
2-(2,6-dichlorophenylamino)-1H-benzimidazol-4-ol;
(1H-benzimidazol-2-yl)-(2-chloro-6-methylphenyl)amine;
(2,6-dichlorophenyl)-(5,6-difluoro-1H-benzimidazol-2-yl)amine;
(2,6-dichlorophenyl)-(4-methyl-1H-benzimidazol-2-yl)amine;
(1H-benzimidazol-2-yl)-(2-chloro-6-fluorophenyl)amine;
(1H-benzimidazol-2-yl)-(2,6-dibromophenyl)amine;
2-(2,6-dichlorophenylamino)-5-fluorobenzimidazole;
2-(2,6-dichlorophenylamino)-4-fluorobenzimidazole;
2-(2-trifluoromethyl-6-chlorophenylamino)benzimidazole;
2-(2,6-dichlorophenylamino)-4,5-difluorobenzimidazole;
2-(2,6-dichlorophenylamino)-5 hydroxybenzimidazole;
2-(2,6-dichlorophenylamino)-4,5,6,7-tetrafluorobenzimidazole; 2-(2,6-dichlorophenylamino)-4,6-difluorobenzimidazole; (1H-benzimidazol-2-yl)-(2-trifluoromethylphenyl)amine;
or
(1H-benzimidazol-2-yl)-(2-bromophenyl)amine;
or their pharmaceutically tolerable salts or trifluoroacetates.

17. A medicament comprising a compound of formula I as claimed in claim 16.

## Revendications

1. Utilisation de composés de formule I 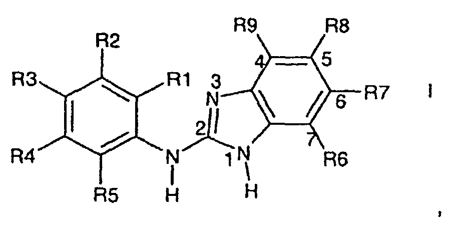 dans laquelle :
R1 et R5 représentent, indépendamment l'un de l'autre, H, F, Cl, Br, I, CN, un groupe alkyle ayant de 1 à 4 atomes de carbone,
qui est non substitué ou partiellement ou totalement substitué par le fluor,
ou
R1 et R5 représentent un groupe cycloalkyle ayant de 3 à 7 atomes de carbone
qui est non substitué ou partiellement ou totalement substitué par le fluor,
ou
R1 et R5 représentent OH, un groupe O-alkyle ayant de 1 à 4 atomes de carbone
qui est non substitué ou partiellement ou totalement substitué par le fluor,
ou
R1 et R5 représentent OCOR10, NR11R12, COR13, COOH, COOR14, CONR11R12, -(O)nSOₘR15,
n est zéro ou 1 ;
m est zéro, 1 ou 2 ;
ou
R1 et R5 représentent un groupe O-phényle
qui est non substitué ou porte de 1 à 3 substituants choisis dans le groupe constitué par F, Cl, Br, I, alkyle ayant de 1 à 4 atomes de carbone, OH, O-alkyle ayant de 1 à 4 atomes de carbone, NR16R17, CN ou alkyl(C₁-C₄)-sulfonyle,
qui est non substitué ou partiellement ou totalement fluoré,
R16 et R17 représentent H ou un groupe alkyle ayant de 1 à 4 atomes de carbone
les groupes alkyle étant non substitués ou partiellement ou totalement fluorés,
R10 représente H ou un groupe alkyle ayant de 1 à 4 atomes de carbone,
qui est non substitué ou partiellement ou totalement substitué par le fluor,
R11 et R12 représentent, indépendamment l'un de l'autre, H, un groupe alkyle ayant de 1 à 4 atomes de carbone, qui peuvent être partiellement ou totalement fluorés, et un groupe CH₂ quelconque peut être remplacé par O ou NR18,
ou R11 et R12 forment ensemble un cycle à 5, 6 ou 7 chaînons ;
ou R11 et R12 représentent COR19 ou SO₂R20 ;
R18, R19 et R20 représentent, chacun indépendamment, H ou un groupe alkyle ayant de 1 à 4 atomes de carbone,
qui est non substitué ou partiellement ou totalement substitué par le fluor ;
R13 et R14 représentent un groupe alkyle ayant de 1 à 4 atomes de carbone,
qui est non substitué ou partiellement ou totalement substitué par le fluor ;
R15 représente un groupe alkyle ou O-alkyle ayant de 1 à 4 atomes de carbone
les groupes alkyle étant non substitués ou partiellement ou totalement fluorés,
ou
R15 représente OH ou NR21R22 ;
R21 et R22 représentent, indépendamment l'un de l'autre, H ou un groupe alkyle ayant de 1 à 4 atomes de carbone, qui est non substitué ou partiellement ou totalement substitué par le fluor, et dans lequel un groupe CH₂ quelconque peut être remplacé par O ou NR23,
R23 représente H ou un groupe alkyle ayant de 1 à 4 atomes de carbone qui est non substitué ou partiellement ou totalement substitué par le fluor ;
ou
R21 et R22 forment ensemble un cycle à 5, 6 ou 7 chaînons ;
R2, R3 et R4 représentent H ou F ;
R6, R7, R8 et R9 représentent, indépendamment les uns des autres, H, F, Cl, Br, I, CN, un groupe alkyle ou O-alkyle ayant de 1 à 4 atomes de carbone,
qui sont non substitués ou partiellement ou totalement substitués par le fluor,
ou R6, R7, R8 et R9 représentent un groupe cycloalkyle ayant de 3 à 7 atomes de carbone,
qui est non substitué ou partiellement ou totalement substitué par le fluor,
ou R6, R7, R8 et R9 représentent OH, OCOR24 ou NR25R26 ;
R24 représente H ou un groupe alkyle ayant de 1 à 4 atomes de carbone,
qui est non substitué ou partiellement ou totalement substitué par le fluor,
R25 et R26 représentent, indépendamment l'un de l'autre représentent H ou un groupe alkyle ayant de 1 à 4 atomes de carbone,
qui est non substitué ou partiellement ou totalement substitué par le fluor,
ou
R25 et R26 représentent COR27 ;
ou R25 et R26 forment ensemble, avec l'atome d'azote auquel ils sont fixés, un cycle à 5, 6 ou 7 chaînons, dans lequel un groupe CH₂ quelconque peut être remplacé par O ou NR18 ;
R27 représente H ou un groupe alkyle ayant de 1 à 4 atomes de carbone,
qui est non substitué ou partiellement ou totalement substitué par le fluor,
ainsi que de leurs sels pharmaceutiquement acceptables,
pour la fabrication d'un médicament destiné au traitement ou à la prophylaxie de troubles de l'impulsion respiratoire, de troubles respiratoires, en particulier de troubles respiratoires dus au sommeil, tels que les apnées du sommeil, du ronflement, de néphropathies aiguës et chroniques, en particulier de l'insuffisance rénale aiguë et de l'insuffisance rénale chronique, de troubles de la fonction intestinale, de troubles de la fonction biliaire, d'états ischémiques du système nerveux central et périphérique et de l'accident vasculaire cérébral, d'états ischémiques des organes périphériques et des membres, d'états de choc, de maladies dans lesquelles la prolifération cellulaire représente une cause primaire ou secondaire, de troubles du métabolisme lipidique, de l'attaque par des ectoparasites en médecine humaine et vétérinaire, pour la fabrication d'un médicament destiné à l'utilisation dans des opérations chirurgicales et des transplantations d'organes et pour la fabrication d'un médicament destiné à la conservation et au stockage de transplants pour des interventions chirurgicales.

2. Utilisation d'un composé I selon la revendication 1, pour la fabrication d'un médicament destiné au traitement ou à la prophylaxie de troubles de l'impulsion respiratoire.

3. Utilisation d'un composé I selon la revendication 1, pour la fabrication d'un médicament destiné au traitement ou à la prophylaxie de troubles respiratoires, en particulier de troubles respiratoires dus au sommeil, tels que les apnées du sommeil.

4. Utilisation d'un composé I selon la revendication 1, pour la fabrication d'un médicament destiné au traitement ou à la prophylaxie du ronflement.

5. Utilisation d'un composé I selon la revendication 1, pour la fabrication d'un médicament destiné au traitement ou à la prophylaxie de néphropathies aiguës et chroniques, en particulier de l'insuffisance rénale aiguë et de l'insuffisance rénale chronique.

6. Utilisation d'un composé I selon la revendication 1, pour la fabrication d'un médicament destiné au traitement ou à la prophylaxie de troubles de la fonction intestinale.

7. Utilisation d'un composé I selon la revendication 1, pour la fabrication d'un médicament destiné au traitement ou à la prophylaxie de la fonction biliaire.

8. Utilisation d'un composé I selon la revendication 1, pour la fabrication d'un médicament destiné au traitement ou à la prophylaxie d'états ischémiques du système nerveux central et périphérique et de l'accident vasculaire cérébral.

9. Utilisation d'un composé I selon la revendication 1, pour la fabrication d'un médicament destiné au traitement ou à la prophylaxie d'états ischémiques des organes périphériques et des membres.

10. Utilisation d'un composé I selon la revendication 1, pour la fabrication d'un médicament destiné au traitement d'états de choc.

11. Utilisation d'un composé I selon la revendication 1, pour la fabrication d'un médicament destiné à l'utilisation dans des opérations chirurgicales et des transplantations d'organes.

12. Utilisation d'un composé I selon la revendication 1, pour la fabrication d'un médicament destiné à la conservation et au stockage de transplants pour des interventions chirurgicales.

13. Utilisation d'un composé I selon la revendication 1, pour la fabrication d'un médicament destiné au traitement de maladies dans lesquelles la prolifération cellulaire représente une cause primaire ou secondaire.

14. Utilisation d'un composé I selon la revendication 1, pour la fabrication d'un médicament destiné au traitement ou à la prophylaxie de troubles du métabolisme lipidique.

15. Utilisation d'un composé I selon la revendication 1, pour la fabrication d'un médicament destiné au traitement de l'attaque par des ectoparasites en médecine humaine et vétérinaire.

16. Composé de formule I 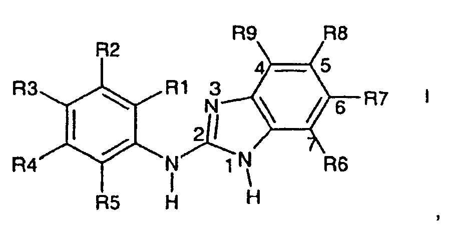 qui est
le 2-(2,6-dichloro-phénylamino)-1H-benzimidazol-4-ol,
la (1H-benzimidazol-2-yl)-(2-chloro-6-méthylphényl)amine,
la (2,6-dichloro-phényl)-(5,6-difluoro-1H-benzimidazol-2-yl)amine,
la (2,6-dichloro-phényl)-(4-méthyl-1H-benzimidazol-2-yl)amine,
la (1H-benzimidazol-2-yl)-(2-chloro-6-fluorophényl)amine,
la (1H-benzimidazol-2-yl)-(2,6-dibromo-phényl)-amine,
le 2-(2,6-dichlorophénylamino)-5-fluorobenzimidazole,
le 2-(2,6-dichlorophénylamino)-4-fluorobenzimidazole,
le 2-(2-trifluorométhyl-6-chlorophénylamino)-benzimidazole,
le 2-(2,6-dichlorophénylamino)-4,5-difluorobenzimidazole,
le 2-(2,6-dichlorophénylamino)-5-hydroxybenzimidazole,
le 2-(2,6-dichlorophénylamino)-4,5,6,7-tétrafluorobenzimidazole,
le 2-(2,6-dichlorophénylamino)-4,6-difluorobenzimidazole,
la (1H-benzimidazol-2-yl)-(2-trifluorométhylphényl) amine,
ou
la (1H-benzimidazol-2-yl)-(2-bromo-phényl)amine, et leurs sels pharmaceutiquement acceptables ou fluoroacétates.

17. Médicament, contenant une quantité efficace d'un composé de formule I selon la revendication 16.
